(19) Europäisches Patentamt
European Patent Office
Office européen des brevets

(11) Veröffentlichungsnummer: **0 158 164**
**A2**

# (12) EUROPÄISCHE PATENTANMELDUNG

(21) Anmeldenummer: 85103091.6

(22) Anmeldetag: 18.03.85

(51) Int. Cl.[4]: C 07 D 311/96
C 07 D 311/22, A 61 K 31/35

(30) Priorität: 31.03.84 DE 3411992

(43) Veröffentlichungstag der Anmeldung: 16.10.85 Patentblatt 85/42

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Kabbe, Hans-Joachim, Dr.
Walter-Flex-Strasse 16
D-5090 Leverkusen 1(DE)

(72) Erfinder: Widdig, Arno, Dr.
Eifgenstrasse 8
D-5068 Odenthal(DE)

(72) Erfinder: Niewöhner, Ulrich, Dr.
Gartenstrasse 3
D-5632 Wermelskirchen 3(DE)

(72) Erfinder: Knorr, Andreas, Dr.
Pahlkestrasse 15
D-5600 Wuppertal 1(DE)

(72) Erfinder: Garthoff, Bernward, Dr.
Händelstrasse 22
D-4010 Hilden(DE)

(72) Erfinder: Kazda, Stanislav, Dr.
Heuselweg 18
D-5600 Wuppertal(DE)

(54) **Substituierte 4-Hydroxy-benzopyrane, Verfahren zu ihrer Herstellung sowie ihre Verwendung in Arzneimitteln.**

(57) Die vorliegende Erfindung betrifft substituierte 4-Hydroxybenzopyrane der Formel I

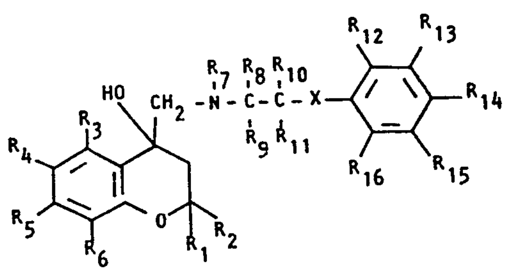

(I)

in welcher $R_1$ bis $R_{16}$ und X die in der Beschreibung angegebene Bedeutung haben, mehrere Verfahren zu ihrer Herstellung sowie ihre Verwendung in Arzneimitteln, insbesondere in kreislaufbeeinflussenden Mitteln.

EP 0 158 164 A2

0158164

BAYER AKTIENGESELLSCHAFT 5090 Leverkusen, Bayerwerk
Konzernverwaltung RP
Patentabteilung KS/bo/c
Ia (Pha)

Substituierte 4-Hydroxy-benzopyrane, Verfahren zu ihrer Herstellung sowie ihre Verwendung in Arzneimitteln

Die vorliegende Erfindung betrifft substituierte 4-Hydroxy-benzopyrane, mehrere Verfahren zu ihrer Herstellung sowie ihre Verwendung in Arzneimitteln, insbesondere in kreislaufbeeinflussenden Mitteln.

Die neuen Verbindungen können durch folgende Formel (I) wiedergegeben werden:

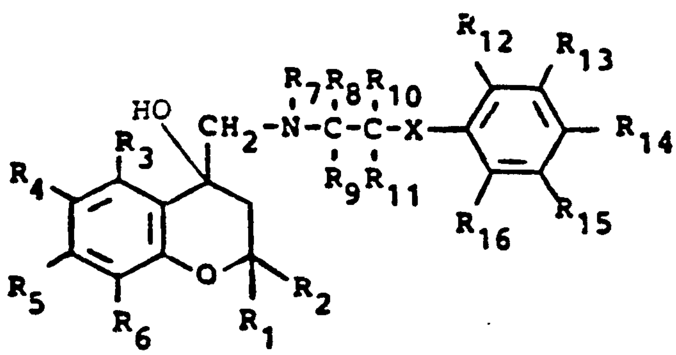

I

in der

$R_1$ und $R_2$ gleich oder verschieden sind und für Wasserstoff, Alkyl, Cycloalkyl, gegebenenfalls substituiertes Aryl, gegebenenfalls substituiertes Aralkyl

stehen, oder in welchen

$R_1$ und $R_2$ zusammen mit dem von ihnen eingeschlossenen Kohlenstoffatom einen carbocyclischen Ring bilden,

$R_3$,$R_4$,$R_5$ und $R_6$ gleich oder verschieden sind und für Wasserstoff, Hydroxy, Halogen, Alkyl, Cycloalkyl, gegebenenfalls substituiertes Aryl, gegebenenfalls substituiertes Aralkyl, Alkoxy, gegebenenfalls substituiertes Aryloxy und gegebenenfalls substituiertes Aralkyloxy stehen,

$R_7$,$R_8$,$R_9$,$R_{10}$ und $R_{11}$ gleich oder verschieden sind und für Wasserstoff oder Alkyl stehen,

X für eine Einfachbindung, für gegebenenfalls durch eine oder zwei Alkylgruppen substituiertes Methylen, für Sauerstoff oder für $NR_{17}$ steht, wobei

$R_{17}$ für Wasserstoff und Alkyl mit bis zu 6 Kohlenstoffatomen steht, oder

$R_{17}$ zusammen mit $R_7$ für $C_2$-$C_3$-Alkylen steht,

$R_{12}$, $R_{13}$, $R_{14}$, $R_{15}$ und $R_{16}$ gleich oder verschieden sind und jeweils für Wasserstoff, Hydroxy, Halogen, Alkyl, Cycloalkyl, Alkoxy, Aralkyloxy, Trifluormethyl, Nitro,

Cyano, die Gruppe 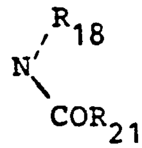 , 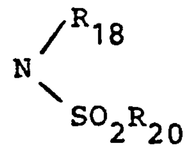 , 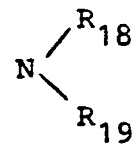 ,

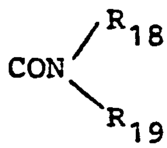 oder 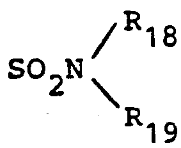 stehen,

wobei $R_{18}$ und $R_{19}$ gleich oder verschieden sind und jeweils für Wasserstoff oder gegebenenfalls substituiertes Alkyl stehen und wobei $R_{20}$ für gegebenenfalls substituiertes Alkyl steht, und wobei $R_{21}$ für Wasserstoff, gegebenenfalls substituiertes Alkyl, gegebenenfalls substituiertes Alkoxy oder gegebenenfalls substituiertes Alkylamino steht,

und

$R_{12}$ und $R_{13}$ oder $R_{13}$ und $R_{14}$ gegebenenfalls gemeinsam jeweils für eine Alkylendioxygruppe oder die Gruppe -CH=CH-CH=CH- stehen,

oder

wobei $R_{18}$ gemeinsam mit $R_{19}$ oder gemeinsam mit $R_{20}$ oder gemeinsam mit $R_{21}$ einen 5- bis 7-gliedrigen heterocyclischen Ring bilden können, der eine Alkylenbrücke mit 2 bis 5 Kohlenstoffatomen und gegebenenfalls noch eine zusätzliche Carbonylgruppe enthält.

Die Erfindung betrifft auch ihre pharmazeutisch unbedenklichen Additionssalze.

Als Alkyl in den Substituenten $R_1$-$R_{21}$ kommen vorzugsweise in Betracht geradkettige oder verzweigte $C_1$-$C_{18}$-Alkylreste, bevorzugt $C_1$-$C_{12}$-Alkylreste, insbesondere $C_1$-$C_6$-Alkylreste, falls im Text nicht anders angegeben.

Als Alkylreste seien beispielhaft genannt:
Methyl, Ethyl, Propyl, Isopropyl, Butyl, Isobutyl, tert.-Butyl, Hexyl, 2-Hexyl, 1,1-Dimethylpentyl, 1,1-Dimethylhexyl, Nonyl, Decyl, Undecyl, Tetradecyl.

Als Cycloalkylreste in den Resten $R_1$, $R_2$, $R_3$, $R_4$, $R_5$, $R_6$, $R_{12}$ bis $R_{16}$ kommen vorzugsweise solche mit 3-18, bevorzugt mit 4-12, insbesondere bevorzugt mit 5 und 6 Kohlenstoffatomen wie beispielsweise Cyclopropyl, Cyclobutyl, Cyclopentyl, Cyclohexyl, Cycloheptyl, Cyclooctyl, Cyclododecyl, Cycloheptadecyl und Cyclooctadecyl, insbesondere bevorzugt Cyclopentyl und Cyclohexyl in Frage.

Als gegebenenfalls substituiertes Alkyl in den Resten $R_{12}$-$R_{21}$ stehen vorzugsweise Alkylgruppen, die 1-, 2- oder 3-fach substituiert sind durch Hydroxy, Halogen, insbesondere Fluor oder Chlor, Cyano, Nitro, Alkoxy mit 1 bis 4 C-Atomen oder Trifluormethoxy.

Als gegebenenfalls substituiertes Aryl in den Resten $R_1$, $R_2$ und $R_3$ bis $R_6$ steht Aryl mit vorzugsweise 6 bis 10 Kohlenstoffatomen im Arylteil. Beispielhaft seien ge-

gebenenfalls substituiertes Phenyl und Naphthyl genannt.

Als gegebenenfalls substituierte Aralkylreste in den Resten $R_1$, $R_2$ und $R_3$ bis $R_6$ kommen vorzugsweise solche mit 7 bis 18 Kohlenstoffatomen, deren aliphatischer Teil 1 bis 8, bevorzugt 1 bis 4 Kohlenstoffatome enthält und deren aromatischer Teil einen carbocyclischen Rest mit 6 bis 10 Kohlenstoffatomen darstellt, in Frage. Beispielhaft seien die folgenden Aralkylreste genannt: Benzyl, Phenylethyl, Phenylpropyl, Phenylbutyl, Naphthylmethyl, bevorzugt Benzyl.

Als Alkoxy in den Resten $R_3$ bis $R_6$ und $R_{12}$ bis $R_{21}$ steht geradkettiges oder verzweigtes Alkoxy mit vorzugsweise 1 bis 6, insbesondere 1 bis 4 Kohlenstoffatomen. Beispielhaft seien Methoxy, Ethoxy, n- und i-Propoxy und n-, i- und t-Butoxy genannt. Als bevorzugte Aryloxygruppen $R_3$ bis $R_6$ und $R_{12}$ bis $R_{16}$ seien solche mit 6 oder 10 Kohlenstoffatomen wie Phenoxy oder Naphthoxy genannt.

Als Aralkoxy in den Resten $R_3$ bis $R_6$ seien solche mit bevorzugt 7 bis 10 Kohlenstoffatomen wie Benzyloxy, Phenylethoxy, Phenylpropoxy, Phenylisopropoxy, Phenylbutoxy und Phenylisobutoxy genannt.

Als Halogene in den Resten $R_3$ bis $R_6$ und $R_{12}$ bis $R_{21}$ seien Fluor, Chlor, Brom und Iod, bevorzugt Fluor, Brom und Chlor genannt.

Falls die Reste $R_1$ und $R_2$ zusammen mit dem durch sie eingeschlossenen Kohlenstoffatom einen carbocyclischen Ring bilden, so kommen 3- bis 12-gliedrige Ringe, bevorzugt 4- bis 7-gliedrige Ringe, in Frage. Als carbocyclische Reste seien beispielsweise genannt: Cyclopropan, Cyclobutan, Cyclopentan, Cyclohexan, Cycloheptan, Cyclononan, Cyclodecan und Cyclododecan.

Als Substituenten der Aryl-, Aralkyl-, Aryloxy- und Aralkyloxy-Reste $R_1$ bis $R_6$ kommen Substituenten in Frage, die unter den Reaktionsbedingungen nicht verändert werden. Beispielsweise seien die Halogene wie Fluor, Chlor, Brom und Iod, die $C_1$-$C_6$-Alkylgruppe, die $C_1$-$C_6$-Alkoxygruppe und die Trifluormethylgruppe genannt.

Als Säuren zur Herstellung der Salze seien beispielsweise genannt: Schwefelsäure, Salzsäure, organische Carbonsäuren wie Äpfelsäure, Citronensäure, Fumarsäure, Maleinsäure, Bernsteinsäure, Essigsäure oder organische Sulfonsäuren wie Naphthalin-1,5-disulfonsäure.

Die Salze der Verbindungen der Formel (I) können in einfacher Weise nach üblichen Salzbildungsmethoden, z.B. durch Lösen der Base und Hinzufügen der Säure erhalten werden und in bekannter Weise, z.B. durch Abfiltrieren, isoliert und gegebenenfalls gereinigt werden.

Bevorzugt stehen in Formel (I):

$R_1$ und $R_2$ gleich oder verschieden für Wasserstoff, $C_1$-$C_6$-Alkyl, $C_5$-$C_7$-Cycloalkyl, Phenyl, gegebenenfalls substituiert (2-fach, insbesondere 1-fach) durch $C_1$-$C_4$-Alkyl, Halogen (Chlor, Brom) und/oder $C_1$-$C_4$-Alkoxy, $C_7$-$C_9$-Aralkyl, dessen Arylrest gegebenenfalls (1- bis 2-fach, insbesondere 1-fach) substituiert ist durch $C_1$-$C_4$-Alkyl, Halogen (Chlor, Brom) und/oder $C_1$-$C_4$-Alkoxy, oder $R_1$ und $R_2$ bilden zusammen mit dem eingeschlossenen C-Atom des Chromanringes einen 4- bis 7-gliedrigen carbocyclischen Ring;

$R_3$ bis $R_6$ gleich oder verschieden für Wasserstoff, Hydroxy, Halogen (Chlor, Brom), $C_1$-$C_6$-Alkyl, $C_5$-$C_7$-Cycloalkyl, Phenyl, gegebenenfalls substituiert (1- bis 2-fach, insbesondere 1-fach) durch $C_1$-$C_4$-Alkyl, Halogen (Chlor, Brom) und/oder $C_1$-$C_4$-Alkoxy, $C_7$-$C_9$-Aralkyl, dessen Arylrest gegebenenfalls (1- bis 2-fach, insbesondere 1-fach) substituiert ist durch $C_1$-$C_4$-Alkyl, Halogen (Chlor, Brom) und/oder $C_1$-$C_4$-Alkoxy;

$R_7$, $R_8$, $R_9$, $R_{10}$, $R_{11}$ gleich oder verschieden sind und für Wasserstoff oder $C_1$-$C_6$-Alkyl stehen,

X für eine Einfachbindung, für Methylen, gegebenenfalls ein- oder zweifach substituiert durch $C_1$-$C_4$-Alkyl, für Sauerstoff oder für $-NR_{17}$ steht, wobei $R_{17}$ Wasserstoff oder $C_1$-$C_4$

Alkyl darstellt oder $R_{17}$ zusammen mit $R_7$ einen $C_2$-Alkylenringschluß bildet,

$R_{12}$, $R_{13}$, $R_{14}$, $R_{15}$ und $R_{16}$ gleich oder verschieden sind und jeweils für Wasserstoff, Hydroxy, Chlor, Fluor, Alkyl mit 1 - 4 C-Atomen, Alkoxy mit 1 bis 3 C-Atomen, Aralkyloxy mit bis zu 8 C-Atomen, Trifluormethyl, Nitro, Cyano,

die Gruppe 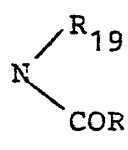 , 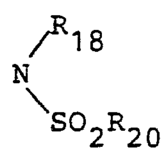 , 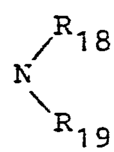 ,

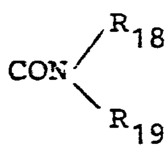 oder 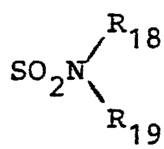 stehen, wobei $R_{18}$ und

$R_{19}$ gleich oder verschieden sind und jeweils für Wasserstoff oder Alkyl mit 1 - 4 C-Atomen stehen, welches gegebenenfalls 1 bis 3 fach substituiert ist durch Halogen,

$R_{20}$ für Alkyl mit 1-6 C-Atomen steht, welches gegebenenfalls 1-3 fach substituiert ist durch Halogen,

$R_{21}$ für Wasserstoff, Alkyl, Alkoxy oder Alkylamino mit jeweils 1-4 C-Atomen pro Alkyl- und Alkoxygruppe steht, wobei der Alkylrest gegebenenfalls 1-3 fach substituiert ist durch Halogen,

0158164

und

$R_{12}$ und $R_{13}$ oder $R_{13}$ und $R_{14}$ gegebenenfalls gemeinsam für eine Alkylendioxygruppe mit 1 oder 2 C-Atomen oder die Gruppe -CH=CH-CH=CH- stehen,

oder

wobei $R_{18}$ gemeinsam mit $R_{19}$ oder gemeinsam mit $R_{20}$ oder gemeinsam mit $R_{21}$ einen 5- oder 6-gliedrigen heterocyclischen Ring bilden kann, der eine Alkylenbrücke mit 2, 3 oder 4 Kohlenstoffatomen enthält und gegebenenfalls noch eine zusätzliche Carbonylgruppe enthält.

Besonders bevorzugt sind Verbindungen der Formel (I), in welcher

$R_1$ und $R_2$ gleich oder verschieden sind und für Wasserstoff, $C_1$-$C_4$-Alkyl stehen oder zusammen mit dem von ihnen eingeschlossenen Kohlenstoffatom einen carbocyclischen $C_5$ oder $C_6$ Ring bilden,

$R_3$ bis $R_6$ gleich oder verschieden sind und Wasserstoff, Hydroxy, $C_1$-$C_4$-Alkyl, $C_1$-$C_4$-Alkoxy oder Chlor bedeuten,

$R_7$ bis $R_{11}$ gleich oder verschieden sind und Wasserstoff oder $C_1$-$C_4$-Alkyl darstellen,

X für eine Einfachbindung, für Sauerstoff, für Methylen oder für $-NR_{17}$ steht, wobei $R_{17}$ Wasserstoff oder $C_1$-$C_3$-Alkyl bedeutet, oder wobei $R_{17}$ zusammen mit $R_7$ einen Ethylenringschluß bildet, und

$R_{12}$ bis $R_{16}$ gleich oder verschieden sind und Wasserstoff, Chlor, Cyclohexyl, $C_1$-$C_4$-Alkyl, $C_1$-$C_3$-Alkoxy, Aralkoxy mit 1-8 C-Atomen, Trifluormethyl, Nitro, Cyano, die Gruppe

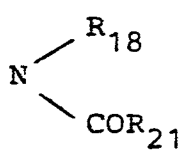 , 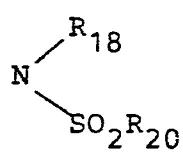 , 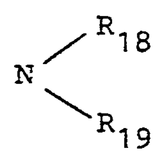 , 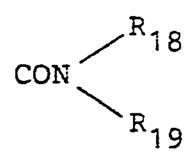 ,

oder 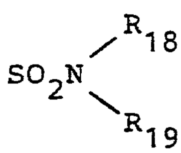 bedeuten, wobei $R_{12}$ und $R_{13}$

oder $R_{13}$ und $R_{14}$ gegebenenfalls zusammen jeweils eine Methylendioxygruppe oder eine -CH=CH-CH=CH-Gruppe bilden und wobei $R_{18}$ und $R_{19}$ gleich oder verschieden sind und jeweils für Wasserstoff oder gegebenenfalls durch Fluor oder Chlor substituiertes Alkyl mit 1-4 C-Atomen stehen und wobei $R_{20}$ für gegebenenfalls durch Fluor oder Chlor substituiertes Alkyl mit 1-4 C-Atomen steht und wobei $R_{21}$ für Wasserstoff, gegebenenfalls durch Fluor oder Chlor substituiertes Alkyl mit 1-4 C-Atomen, Alkoxy oder Alkylamino mit jeweils 1-2 C-Atomen steht.

Als neue Verbindungen der allgemeinen Formel (I) seien beispielhaft genannt:

4-[N-(2-Phenylethyl)-aminomethyl]-4-hydroxy-2,2-spirocyclopentachroman

4-{N-[2-(3-Chlorphenyl)-ethyl]-aminomethyl}-4-hydroxy-2,2-spirocyclohexachroman

4-{N-[2-(4-Methylphenyl)-ethyl]-aminomethyl}-4-hydroxy-2,2-dimethylchroman

4-{N-[2-(3,4-Methylendioxyphenyl)-ethyl]-aminomethyl}-4-hydroxy-2,2-spirocyclopentachroman

4-{N-[3-(3,4-Dimethoxyphenyl)-propyl]-aminomethyl}-4-hydroxy-6,7-dimethyl-2,2-spirocyclopentachroman

4-{N-[3-(4-Trifluormethylphenyl)-propyl]-aminomethyl}-4-hydroxy-6-methoxy-chroman

4-{N-[2-(3,4-Dichlorphenoxy)-ethyl]-aminomethyl}-4-hydroxy-2,2-spirocyclohexachroman

4-{N-[2-(3,4-Dimethoxyphenoxy)-ethyl]-aminomethyl}-4-hydroxy-2,2-spirocyclopentachroman

4-{N-[2-(3,4-Methylendioxyphenoxy)-isopropyl]-aminomethyl}-4-hydroxy-7-chlor-2,2-dimethylchroman

4-{N-[4-(2-Methylphenyl)-butyl]-aminomethyl}-4-hydroxy-2,2-spirocyclohexachroman

4-{N-[4-(Pentafluorophenyl)-butyl]-aminomethyl}-4-hydroxy-2,2-spirocyclopentachroman

4-{N-[4-(3-Chlor-5-methylphenyl)-butyl]-aminomethyl}-4-hydroxy-6,8-dimethylchroman

4-{N-[4-(3,4-Ethylendioxyphenyl)-butyl]-aminomethyl}-4-hydroxy-2,2-spirocyclopentachroman

4-{N-[4-(2-Naphthyl)-butyl]-aminomethyl}-4-hydroxy-2,2-spirocyclopentachroman

4-{N-[4-(3,5-Dimethyl-4-methoxyphenyl)-butyl]-aminomethyl}-4-hydroxy-2,2-dimethylchroman

4-{N-[4-(3-Carbomethoxy-5-methoxyphenyl)-butyl]-aminomethyl}-4-hydroxy-2,2-spirocyclohexachroman

4-{N-[4-(3-Methoxy-5-carbamoylphenyl)-butyl]-aminomethyl}-4-hydroxy-2,2-spirocyclopentachroman

4-{N-[4-(3,4-Methylendioxy-5-carbamoylphenyl)-butyl]-aminomethyl}-4-hydroxy-2,2-spirocyclopentachroman

4-{N-[4-(3-Methyl-5-aminosulfonylphenyl)-butyl]-aminomethyl}-4-hydroxy-6,7-dimethyl-2,2-spirocyclohexachroman

4-{N-[4-(3-Chlor-5-aminosulfonylphenyl)-butyl]-aminomethyl}-4-hydroxy-2,2-dimethylchroman

4-{N-[4-(3,4-Methylendioxy-5-aminosulfonylphenyl)-butyl]-aminomethyl}-4-hydroxy-chroman

4-{N-[4-(3-Aminophenyl)-butyl]-aminomethyl}-4-hydroxy-6-methoxy-2,2-spirocyclopentachroman

4-{N-[4-(3-N,N-Dimethylaminophenyl)-butyl]-aminomethyl}-4-hydroxy-2,2-spirocyclopentachroman

4-{N-[4-(3-Succinimidophenyl)-butyl]-aminomethyl}-4-hydroxy-2,2-spirocyclopentachroman

4-{N-[4-(4-Methylsulfonylaminophenyl)-butyl]-aminomethyl}-4-hydroxy-2,2-spirocyclopentachroman

4-{N-[4-(3-Ethylsulfonylaminophenyl)-butyl]-aminomethyl}-4-hydroxy-2,2-spirocyclopentachroman

4-{N-[4-(3-Acetylaminophenyl)-butyl]-aminomethyl}-4-hydroxy-2,2-spirocyclopentachroman

4-{N-[4-(4-Cyanophenyl)-butyl]-aminomethyl}-4-hydroxy-7-chlor-2,2-spirocyclopentachroman

4-{N-[4-(3-Pyrrolidinophenyl)-butyl]aminomethyl}-4-hydroxy-2,2-dimethylchroman

4-{N-[1-(3-Nitrophenoxy)-isopropyl]-aminomethyl}-4-hydroxy-6,8-dimethyl-chroman

4-{N-[1-(3-Aminophenoxy)-isopropyl]-aminomethyl}-4-hydroxy-6,8-dimethyl-chroman

4-{N-[1-(3-Methylsulfonylphenoxy)-isopropyl]-aminomethyl}-4-hydroxy-6,8-dimethyl-chroman

4-{N-[1-(3-N,N-Dimethylaminosulfonylphenoxy)-isopropyl]-aminomethyl}-4-hydroxy-2,2-spirocyclopentachroman

4-{N-[1-(4-Carbamoylphenoxy)-isopropyl]-aminomethyl}-4-hydroxy-6,7-dimethyl-2,2-spirocyclohexachroman

4-{N-[4-(2-Nitrophenyl)-butyl]-3-aminomethyl}-4-hydroxy-2,2-spirocyclopentachroman

4-{N-[4-(3-Methoxy-4-hydroxy-5-nitrophenyl)-butyl]-aminomethyl}-4-hydroxy-2,2-spirocyclopentachroman

4-{N-[4-(3-Chlor-5-nitrophenyl)-butyl]-aminomethyl}-4-hydroxy-2,2-dimethylchroman

4-{N-[4-(3-Methyl-5-nitrophenyl)-butyl]-aminomethyl}-4-hydroxy-6,7-dimethyl-2,2-spirocyclopentachroman

4-{N-[4-(4-Methtylsulfonylaminophenyl)-butyl]-aminomethyl}-4-hydroxy-6,7-dimethyl-2,2-spirocyclopentachroman

4-{N-[4-(3-Methoxy-5-methylsulfonylaminophenyl)-butyl]-aminomethyl}-4-hydroxy-2,2-spirocyclopentachroman

4-{N-[4-(3-Methyl-5-acetylaminophenyl)-butyl]-aminomethyl}-4-hydroxy-2,2-spirocyclopentachroman

4-{N-[1-(4-Hydroxyphenyl)-2-methyl-isopropyl]-aminomethyl}-4-hydroxy-2,2-spirocyclopentachroman

4-{N-[1-(3,5-Dimethyl-4-methoxyphenyl)-2-methyl-isopropyl]-aminomethyl}-4-hydroxy-2,2-spirocyclopentachroman

4-{N-[1-(3-Nitro-4-methoxyphenyl)-2-methyl-isopropyl]-aminomethyl}-4-hydroxy-2,2-spirocyclopentachroman

4-{N-[1-(3-Methylsulfonylamino-4,5-dimethoxyphenyl)-2-methyl-isopropyl]-aminomethyl}-4-hydroxy-2,2-spirocyclopentachroman

Die Erfindung betrifft weiterhin verschiedene Verfahren zur Herstellung der Verbindungen der Formel (I).

Man setzt entweder

A) Amine der Formel (II)

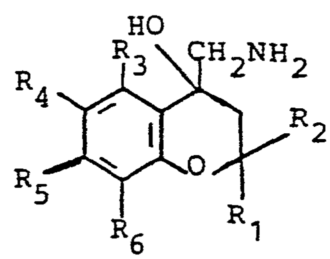

(II)

in welcher

$R_1, R_2, R_3, R_4, R_5$ und $R_6$ die oben angegebene Bedeutung haben,

mit Carbonylverbindungen der Formel (III)

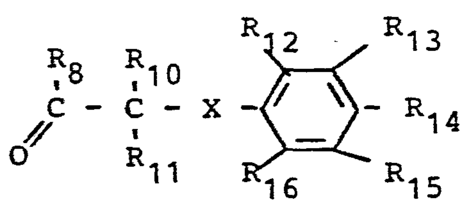

(III)

in welcher

$R_8$ bis $R_{16}$ die oben angegebene Bedeutung haben, mit der Maßgabe, daß X nicht für $NR_{17}$ steht,

in Gegenwart von reduzierenden Agentien um,

oder

B) Verbindungen der Formel (IV)

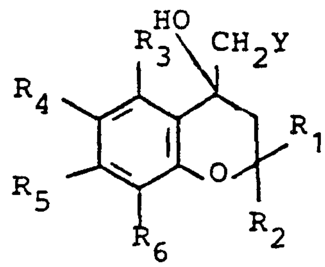

(IV)

gegebenenfalls nach Isolierung der durch Abspaltung von HY entstehenden Verbindungen der Formel (IVa)

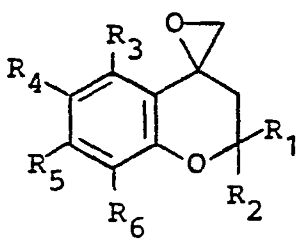

(IVa)

wobei in den Formeln (IV) und (IVa)

$R_1$, $R_2$, $R_3$, $R_4$, $R_5$ und $R_6$ die oben angegebene Bedeutung haben, und

Y für nucleophil austauschbare Gruppen, wie Alkyl- oder Aryl substituierte Sulfonyloxygruppen, Brom oder Chlor steht,

mit Aminen der Formel (V)

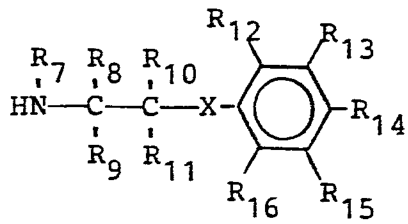 (V)

wobei $R_7$ bis $R_{16}$ die oben angegebene Bedeutung haben,

in Gegenwart eines säurebindenden Mittels um.

Als reduzierende Agentien, wie sie in der Verfahrensvariante A eingesetzt werden können, seien beispielhaft komplexe Metallhydride genannt. Bevorzugt sind Alkaliborhydride, Alkalicyanoborhydride und/oder Alkalialanate, insbesondere Natrium- oder Lithiumverbindungen. Namentlich genannt seien Natriumborhydrid, Natriumcyanoborhydrid oder Lithiumalanat. Ebenso kann auch katalytisch erregter Wasserstoff bei erhöhten Drucken und Temperaturen eingesetzt werden.

Die reduzierenden Agentien können in äquivalenten Mengen bis zum Überschuß von 100 %, bevorzugt von äquivalenten Mengen bis zum Überschuß von 20 %, bezogen auf die eingesetzte Carbonylverbindung, eingesetzt werden.

Die in der Herstellungsvariante B eingesetzten säurebindenden Mittel sind bekannte Basen. Beispielhaft seien genannt: Erdalkali- oder Alkalihydroxide wie Natrium- und/oder Kaliumhydroxid, Erdalkali- oder Alkalicarbonate wie Natriumbicarbonat oder Kaliumcarbonat,

organische Stickstoffbasen wie Triethylamin, Tributylamin oder Benzyltrimethylammoniumhydroxid.

Diese säurebindenden Mittel können in äquivalenten Mengen bis zum Überschuß von 100 %, bevorzugt in äquivalenten Mengen bis zum Überschuß von 20 %, bezogen auf die eingesetzte Halogenverbindung, eingesetzt werden.

Die erfindungsgemäßen Reaktionen werden in Lösungsmitteln durchgeführt. Als Lösungsmittel kommen alle für die jeweilige Reaktion inerten Lösungsmittel in Frage, vorzugsweise seien genannt:

Alkohole wie Methanol, Ethanol, Isopropanol oder tert.-Butanol, Ether wie Diethylether, Diisopropylether, Tetrahydrofuran oder Dioxan, Kohlenwasserstoffe wie Hexan, Cyclohexan, Benzol oder Toluol, Chlorkohlenwasserstoffe wie Methylenchlorid, Chloroform, Tetrachlorkohlenstoff oder Chlorbenzol, ferner Acetonitril, Dimethylformamid, Dimethylsulfoxid oder Mischungen solcher Lösungsmittel.

Die Reaktionstemperaturen können in einem größeren Bereich variiert werden. Im allgemeinen arbeitet man zwischen -50 und 150°C, vorzugsweise von -10 bis 120°C.

Bei der Durchführung der erfindungsgemäßen Verfahren setzt man bevorzugt bezogen auf 1 Mol der 4-Hydroxychromanverbindungen der Formel (II) oder der Formeln (IV) bzw. (IVa) 0,5 bis 2 Mole des Amins der Formel (V) oder der Carbonylverbindung der Formel (III) um.

Besonders bevorzugt beträgt das Molverhältnis der Reaktionspartner 1:1. Benutzt man einen Überschuß, so setzt man bevorzugt einen Überschuß an Amin der Formel (V) oder an Carbonylverbindung der Formel (III) ein.

Die Reaktionsprodukte können durch Destillation, Auskristallisieren, Einengen und Umlösen oder chromatographische Trennung gewonnen werden.

Die bei der Herstellung der erfindungsgemäßen Verbindungen eingesetzten Amine der Formel (V) sind zum Teil bekannt (vgl. z.B. Arch. Pharm. 316, 193 (1983)) bzw. können nach analogen Verfahren erhalten werden.

Als Beispiele für die Amine der Formel (V) seien genannt:
1-(4-Hydroxyphenyl)-2-methyl-isopropylamin, 1-(2-Methoxy-4-hydroxyphenyl)-2-methyl-isopropylamin, 1-(2,6-Dimethyl-4-hydroxyphenyl)-2-methyl-isopropylamin, 1-(2-Chlor-4-hydroxyphenyl)-2-methyl-isopropylamin, 1-(3,5-Dimethyl-4-methoxyphenyl)-2-methyl-isopropylamin, 1-(3,4,5-Trimethoxyphenyl)-2-methyl-isopropylamin, 1-(2,6-Dichlor-4-methoxyphenyl)-2-methyl-isopropylamin, 1-(3,5-Dichlor-4-methoxy-phenyl)-2-methyl-isopropylamin,

2-Amino-4-(3-nitrophenyl)-butan, 2-Amino-4-(3-methylsulfonylaminophenyl)-butan, 2-Amino-4-(4-methylsulfonylaminophenyl)-butan, 2-Amino-4-(3-acetaminophenyl)-butan, 2-Amino-4-(2-nitrophenyl)-butan, 2-Amino-4-(3-N,N-dimethylaminosulfonylphenyl)-butan, 2-Amino-4-(3-N-methylcarbamoylphenyl)-butan, 2-Amino-4-(3,4-dimethoxy-5-methylsulfonylaminophenyl)-butan.

Die bei der Herstellung der erfindungsgemäßen Verbindungen eingesetzten 4-Hydroxy-chromanamine der Formel (II) sind teilweise bekannt (J. med. Chem. 1982, 393), oder können nach analogen Verfahrensweisen dargestellt werden.

Beispielhaft seien folgende Verfahrenswege schematisch angegeben, ausgehend von Chroman-4-onen:

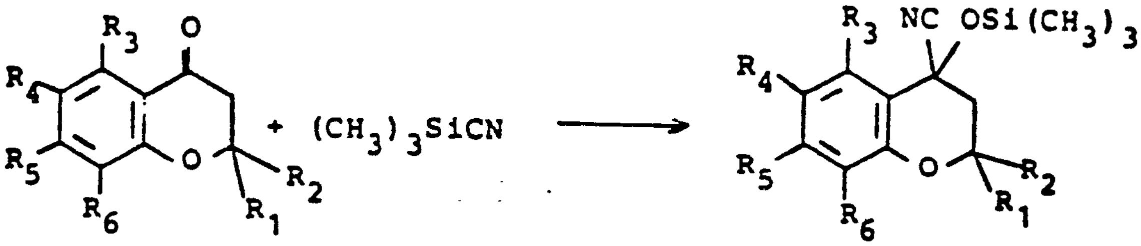

Die hierbei entstehenden 4-Cyan-4-trimethylsilyloxychromane können mit Lithiumaluminiumhydrid zu 4-Aminomethyl-4-hydroxychromanen hydriert werden, wie in folgendem Schema angegeben:

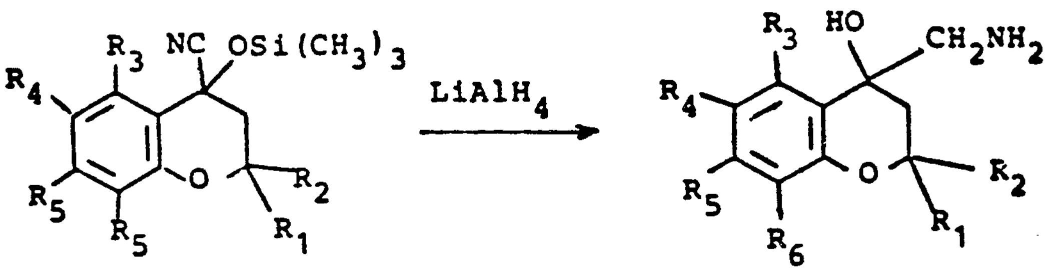


Als Beispiele für die 4-Hydroxy-4-aminomethylchromane (II) seien genannt:

4-Aminomethyl-4-hydroxy-chroman,
4-Aminomethyl-4-hydroxy-2-methyl-chroman,
4-Aminomethyl-4-hydroxy-2,2-dimethyl-chroman,
4-Aminomethyl-4-hydroxy-2-propyl-chroman,
4-Aminomethyl-4-hydroxy-2-isopropyl-chroman,
4-Aminomethyl-4-hydroxy-2,2-diethyl-chroman,
4-Aminomethyl-4-hydroxy-2-methyl-2-propyl-chroman,
4-Aminomethyl-4-hydroxy-2-hexyl-chroman,
4-Aminomethyl-4-hydroxy-2-cyclopentyl-chroman,
4-Aminomethyl-4-hydroxy-2-cyclohexyl-chroman,
4-Aminomethyl-4-hydroxy-2-spirocyclopenta-chroman,
4-Aminomethyl-4-hydroxy-2-spirocyclohexa-chroman,
4-Aminomethyl-4-hydroxy-6-methyl-2-spirocyclopenta-chroman,
4-Aminomethyl-4-hydroxy-7-methyl-2-spirocyclopenta-chroman,
4-Aminomethyl-4-hydroxy-6,8-dimethyl-2-spirocyclopenta-chroman,
4-Aminomethyl-4-hydroxy-6-chlor-2-spirocyclopenta-chroman,
4-Aminomethyl-4-hydroxy-6-methoxy-2-spirocyclopenta-chroman,
4-Aminomethyl-4-hydroxy-7-methoxy-2-spirocyclopenta-chroman,
4-Aminomethyl-4-hydroxy-8-isopropoxy-2-spirocyclopenta-chroman,
4-Aminomethyl-4-hydroxy-7-phenoxy-2-spirocyclopenta-chroman,
4-Aminomethyl-4-hydroxy-7-benzyloxy-2-spirocyclopenta-chroman,
4-Aminomethyl-4-hydroxy-7-phenyl-2-spirocyclopenta-chroman,

4-Aminomethyl-4-hydroxy-6-methyl-2-spirocyclohexachroman, 4-Aminomethyl-4-hydroxy-6-chlor-2-spirocyclohexachroman, 4-Aminomethyl-4-hydroxy-7-methoxy-2-spirocyclohexachroman, 4-Aminomethyl-4-hydroxy-6-methyl-2,2-dimethylchroman, 4-Aminomethyl-4-hydroxy-7-methoxy-2,2-dimethylchroman.

Die bei der Herstellung der erfindungsgemäßen Verbindungen verwendeten Carbonylverbindungen der Formel (III) sind bekannt (vgl. z.B. Beilsteins Handbuch der organischen Chemie 6, 151, II 152; 7, 292, 303, 304, 314, I 154, 161, 162, 167, II 226, 233, 236, 243) oder können nach analogen Verfahren hergestellt werden.

Als Beispiele für die Carbonylverbindungen (III) seien genannt:

Phenylacetaldehyd, 3-Chlorphenylacetaldehyd, 4-Methylphenylacetaldehyd, 4-Isopropylphenylacetaldehyd, 3-Methoxy-phenylacetaldehyd, 3,4-Dimethoxyphenylacetaldehyd, 3,4-Methylendioxyphenylacetaldehyd, α-Phenylpropionaldehyd, ß-Phenylpropionaldehyd, ß-(4-Chlorphenyl)-propionaldehyd, ß-(4-Trifluormethylphenyl)-propionaldehyd, ß-(3,4-Dimethoxyphenyl)-propionaldehyd, ß-(3,4-Methylendioxyphenyl)-propionaldehyd, Phenoxy-acetaldehyd, 4-Chlorphenoxyacetaldehyd, 3,4-Dimethylphenoxyacetaldehyd, 3,4-Dimethoxyphenoxyacetaldehyd, Phenylaceton,3,4-Dimethoxyphenylaceton, 3,4-Methylendioxyphenylaceton, Benzylaceton,

4-(3-Methylphenyl)-butanon-2; 4-(4-Methylphenyl)-butanon-2; 4-(2-Methylphenyl)-butanon-2; 4-(3-Ethylphenyl)-butanon-2; 4-(3-Methyl-5-tert.-butylphenyl)-butanon-2; 4-(3,5-Di-tert.-butylphenyl)-butanon-2; 4-(2,4-Dimethylphenyl)-butanon-2; 4-Pentafluorphenyl-butanon-2; 4-(3-Chlor-5-methylphenyl)-butanon-2; 4-(2-Chlor-3-methylphenyl)-butanon-2; 4-(2,3,4-Trimethoxyphenyl)-butanon-2; 4-(3,4,5-Trimethoxyphenyl)-butanon-2; 4-(3,5-Di-isopropoxyphenyl)-butanon-2; 4-(3-Chlor-5-methoxyphenyl)-butanon-2; 4-(3,4-Methylendioxyphenyl)-butanon-2; 4-(3,4-Ethylendioxyphenyl)-butanon-2; 4-(2-Naphthyl)-butanon-2; 4-(3-Methoxy-5-tert.-butylphenyl)-butanon-2; 4-(3-Trifluormethylphenyl)-butanon-2; 4-(3-Benzyloxyphenyl)-butanon-2; 4-(2-Methyl-4-brom-5-methoxyphenyl)-butanon-2; 4-(3,5-Dimethyl-4-methoxyphenyl)-butanon-2; 4-(4-Hydroxyphenyl)-butanon-2; 4-(3-Carbomethoxy-5-methoxyphenyl)-butanon-2; 4-(3-Carbamoylphenyl)-butanon-2; 4-(4-Carbamoylphenyl)-butanon-2; 4-(4,5-Dimethoxy-3-carbamoylphenyl)-butanon-2; 4-(3-Methoxy-5-carbamoylphenyl)-butanon-2; 4-(3-Methyl-5-carbamoylphenyl)-butanon-2; 4-(3-Trifluormethyl-5-carbamoylphenyl)-butanon-2; 4-(2,4-Dichlor-5-carbamoylphenyl)-butanon-2; 4-(3,4-Methylendioxy-5-carbamoylphenyl)-butanon-2;

4-(3-Aminosulfonylphenyl)-butanon-2; 4-(4-Aminosulfonylphenyl)-butanon-2; 4-(4,5-Dimethoxy-3-aminosulfonylphenyl)-butanon-2; 4-(3-Methoxy-5-aminosulfonylphenyl)-butanon-2; 4-(3-Methyl-5-aminosulfonylphenyl)-butanon-2; 4-(3-Trifluormethyl-5-aminosulfonylphenyl)-butanon-2; 4-(3-Chlor-5-aminosulfonylphenyl)-butanon-2; 4-(3,4-Methylendioxy-5-aminosulfonylphenyl)-butanon-2;

4-(3-Aminophenyl)-butanon-2; 4-(3-N-Methylaminophenyl)-butanon-2; 4-(3-N,N-Dimethylaminophenyl)-butanon-2; 4-(3-N-Succinimidophenyl)-butanon-2; 4-(3-Ethylsulfonylaminophenyl)-butanon-2; 4-(3-n-Butylsulfonylaminophenyl)-butanon-2; 4-(3-N-Methyl-methylsulfonylaminophenyl)-butanon-2; 4-(3-Propionylaminophenyl)-butanon-2; 4-(3-N-Methyl-acetylaminophenyl)-butanon-2; 4-(4-Cyanophenyl)-butanon-2; 4-(3-Pyrrolidinophenyl)-butanon-2; 1-(4-Nitrophenoxy)-propanon-2; 1-(3-Nitrophenoxy)-propanon-2; 1-(3-Aminophenoxy)-propanon-2; 1-(3-Methylsulfonylaminophenoxy)-propanon-2; 1-(4-N-Methyl-methylsulfonylaminophenoxy)-propanon-2; 1-(3-N-Acetylaminophenoxy)-propanon-2; 1-(3-N,N-Dimethylaminosulfonylphenoxy)-propanon-2; 1-(4-Aminosulfonylphenoxy)-propanon-2; 1-(4-Carbamoyl-phenoxy)-propanon-2;

4-(3-Nitrophenyl)-butanon-2; 4-(4-Nitrophenyl)-butanon-2; 4-(2-Nitrophenyl)-butanon-2; 4-(4,5-Dimethoxy-3-nitrophenyl)-butanon-2; 4-(3-Methoxy-5-nitrophenyl)-butanon-2; 4-(3-Methyl-5-nitrophenyl)-butanon-2; 4-(3-Trifluormethyl-5-nitrophenyl)-butanon-2; 4-(3-Methoxy-4-hydroxy-5-nitrophenyl)-butanon-2; 4-(3-Chlor-5-nitrophenyl)-butanon-2; 4-(3,4-Methylendioxy-5-nitrophenyl)-butanon-2;

4-(3-Methylsulfonylaminophenyl)-butanon-2; 4-(4-Methylsulfonylaminophenyl)-butanon-2; 4-(4,5-Dimethoxy-3-methylsulfonylaminophenyl)-butanon-2; 4-(3-Methoxy-5-methylsulfonylaminophenyl)-butanon-2; 4-(3-Methyl-5-methylsulfonylaminophenyl)-butanon-2; 4-(3-Trifluormethyl-5-methylsulfonylaminophenyl)-butanon-2; 4-(3,5-bis-Methylsulfonylaminophenyl)-butanon-2; 4-(3-Methoxy-4-hydroxy-5-methylsulfonylaminophenyl)-butanon-2; 4-(3,4-Methylendioxy-5-methylsulfonylaminophenyl)-butanon-2; 4-(3-Acetylaminophenyl-butanon-2; 4-(4-Acetylaminophenyl)-butanon-2; 4-(4,5-Dimethoxy-3-acetylaminophenyl)-butanon-2; 4-(3-Methoxy-5-acetylaminophenyl)-butanon-2; 4-(3-Methyl-5-acetylaminophenyl)-butanon-2; 4-(3-Trifluormethyl-5-acetylaminophenyl)-butanon-2; 4-(3,4-Methylendioxy-5-acetylaminophenyl)-butanon-2.

Die bei der Herstellung der erfindungsgemäßen Verbindungen gemäß Variante B eingesetzten Verbindungen der Formeln (IV) und (IVa) sind bisher nicht bekannt. Sie können nach bekannten Methoden hergestellt werden, z.B. durch Hydrolyse der bekannten 4-Cyano-4-trimethylsilyloxy-chromane (vgl. J. med. Chem. 1982, 393), zur α-Hydroxycarbonsäure bzw. -ester, anschließender Reduktion zum Diol und Umsetzung zu Verbindungen der Formel (IV) bzw. (IVa).

Die erfindungsgemäßen 4-Hydroxy-chromanderivate zeigen überraschenderweise eine antihypertensive Wirkung und können deshalb in freier Form oder in Form ihrer pharmazeutisch unbedenklichen Säureadditionssalze als Arzneimittel eingesetzt werden.

Die neuen Verbindungen haben ein breites und vielseitiges pharmakologisches Wirkungsspektrum und überraschend lange Wirkungsdauer.

Im einzelnen konnten im Tierexperiment folgende Hauptwirkungen nachgewiesen werden:

1. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten manifestieren. Die Verbindungen eignen sich daher besonders als Cerebraltherapeutika.

2. Die Verbindungen senken den Blutdruck von normotonen und hypertonen Tieren und können somit als antihypertensive Mittel verwendet werden.

Die erfindungsgemäßen Verbindungen eignen sich insbesondere zur Prophylaxe und Heilung von akuten und chronischen Herzkrankheiten, zur Therapie des Hochdrucks sowie zur Behandlung von cerebralen und peripheren Funktions- und Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und dem individuellen Verhalten gegenüber dem Medikament bzw. der Art von dessen Formulierung und dem Zeipunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungspielraum vorgesehen. Sinngemäß gelten hierbei auch die obigen Ausführungen.

Erfindungsgemäße Beispiele

Herstellung nach Variante A

Beispiel 1

Ein Gemisch von 7 g 2,2-Spirocyclopenta-4-hydroxy-4-aminomethylchroman, 6,5 g 4-(m-Trifluormethylphenyl)-butanon-(2) und 70 ml Toluol wird 1 Std. am Wasserabscheider auf 115°C erwärmt. Anschließend wird die Lösung eingeengt, mit 150 ml abs. Tetrahydrofuran verdünnt und portionsweise bei 20°C mit 5 g Lithiumaluminiumhydrid versetzt. Nach 4-stündigem Rühren bei Rückflußtemperatur wird abgekühlt und vorsichtig bei ca. 0°C mit 5 ml Wasser und 15 ml 15-proz. Kalilauge verrührt. Danach saugt man ab, engt das Filtrat ein und destilliert den Rückstand bei 250°C/0,03 torr. Das Kernresonanzspektrum stimmt mit der Struktur des 4-{N-/4-(3-Trifluormethylphenyl)-2-butyl7-aminomethyl}-4-hydroxy-2,2-spirocyclopentachromans überein. Ausbeute: 68 % der Theorie.

Beispiel 2

Man arbeitet wie in Beispiel 1, verwendet als Keton jedoch 7,5 g 4-(m-Trifluormethyl-p-chlorphenyl)-butanon-(2). Nach der Wasserabspaltung wird die Toluol-Lösung des intermediär gebildeten Imins eingeengt, der Rückstand in 150 ml Methanol gelöst und diese Lösung in 2 Std. mit 5 g Natriumborhydrid versetzt. Nach 2 Tagen

wird die Lösung eingeengt und mit Wasser und Toluol (je 150 ml) versetzt. Die organische Phase wird eingeengt und im Kugelrohr destilliert. Man erhält 9,5 g 4-{N-[4-(3-Trifluormethyl-4-chlorphenyl)-2-butyl]-aminomethyl}-4-hydroxy-2,2-spirocyclopentachroman. Sdp. 250°/0,04 torr. Ausbeute: 62 % der Theorie.

In analoger Weise wurden hergestellt:

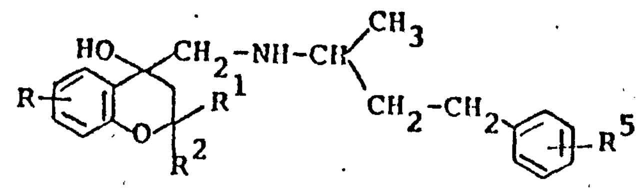

| Beispiel Nr. | R | $R^1/R^2$ | $R^5$ | Sdp. |
|---|---|---|---|---|
| 3 | H | $-(CH_2)_4-$ | $3,5-(CF_3)_2$ | 230-250°C/0,05 torr |
| 4 | H | $-(CH_2)_4-$ | $3,5-(CH_3O)_2$ | 250-260°C/0,05 torr |
| 5 | H | $-(CH_2)_4-$ | $3,5-(CH_3)_2$ | 240-250°C/0,02 torr |
| 6 | H | $-(CH_2)_4-$ | $3-CH_3/5-t-C_4H_9$ | 250°C/0,03 torr |
| 7 | H | $-(CH_2)_5-$ | $3-CH_3/5-t-C_4H_9$ | 240°C/0,02 torr |
| 8 | H | $-(CH_2)_5-$ | $3,4-O-CH_2-O$ | 250°C/0,02 torr |
| 9 | $6,7-(CH_3)_2$ | $-(CH_2)_4-$ | $3,5-(CH_3O)_2$ | 260-70°C/0,02 torr |
| 10 | $6,7-(CH_3)_2$ | $-(CH_2)_4-$ | $3,4-O-CH_2-O$ | 250-60°C/0,02 torr |
| 11 | $6,7-(CH_3)_2$ | $-(CH_2)_4-$ | $3-CH_3/5-t-C_4H_9$ | 260°C/0,02 torr |
| 12 | $6,7-(CH_3)_2$ | $-(CH_2)_4-$ | $3-CF_3$ | 250°C/0,04 torr |
| 13 | H | $-(CH_2)_4-$ | $3,4-O-CH_2-O$ | 240-50°C/0,04 torr |
| 14 | H | $-(CH_2)_5-$ | $3,4,5-(CH_3O)_3$ | 270°C/0,02 torr |
| 15 | $6,7-(CH_3)_2$ | $-(CH_2)_4-$ | $3,4,5-(CH_3O)_3$ | 275°C/0,02 torr |
| 16 | H | $-(CH_2)_4-$ | $3,4,5-(CH_3O)_3$ | 260-70°C/0,03 torr |
| 17 | $6-CH_3O$ | $-(CH_2)_4-$ | $3-CF_3$ | 250°C/0,05 torr |
| 18 | 7-Cl | $(CH_3)_2$ | $3-CH_3/5-t-C_4H_9$ | 240°C/0,03 torr |
| 19 | $6,8-(CH_3)_2$ | $(CH_3)_2$ | $3-CF_3$ | 230°C/0,02 torr |

| Beispiel Nr. | R | $R^1/R^2$ | $R^5$ | Sdp. (Schmelzpunkt) |
|---|---|---|---|---|
| 20 | H | $-(CH_2)_4-$ | $3-SO_2(CH_3)_2$ | Öl 1) |
| 21 | H | $-(CH_2)_4-$ | $3-CONHCH_3$ | Öl 1) |
| 22 | $6,7-(CH_3)_2$ | $-(CH_2)_5-$ | $3-CONH_2$ | Öl 1) |
| 23 | H | $(CH_3)_2$ | $3-SO_2NH_2$ | Öl 1) |
| 24 | H | $-(CH_2)_4-$ | $3-SO_2NH_2/4-CH_3O$ | Öl 1) |
| 25 | H | $-(CH_2)_4-$ | $3-NO_2$ | 225-235°C/0,04 mmHg |
| 26 | H | $-(CH_2)_4-$ | $3-NO_2/5-OCH_3$ | Öl 1) |
| 27 | H | $-(CH_2)_4-$ | $4-NO_2$ | 250°C/0,03 mmHg |
| 28 | H | $-(CH_2)_4-$ | $2-NO_2/3,4-O-CH_2-O-$ | (172-175°C (Fumarat)) |
| 29 | H | $-(CH_2)_4-$ | $3-NH_2$ | (119-123°C (Maleinat)) |
| 30 | H | $-(CH_2)_4-$ | $3-NH-COCH_3$ | Öl 1) |
| 31 | H | $-(CH_2)_4-$ | $3-N(CH_3)COCH_3$ | Öl 1) |
| 32 | H | $-(CH_2)_4-$ | $3-NHSO_2CH_3$ | (218-221°C (Hydrochlorid)) |
| 33 | H | $-(CH_2)_4-$ | $3-NHSO_2n-C_4H_9$ | (119-126°C (Fumarat) |
| 34 | H | $-(CH_2)_4-$ | $3-NHSO_2CH_3/5-OCH_3$ | Öl 1) |
| 35 | H | $-(CH_2)_4-$ | $2-NHSO_2CH_3/4,5-O-CH_2-O-$ | (149-151°C (Maleinat)) |
| 36 | H | $-(CH_2)_4-$ | $3-N(CH_3)SO_2CH_3$ | (169-171°C (Fumarat)) |
| 37 | H | $-(CH_2)_4-$ | $3-CH_3/5-NHSO_2CH_3$ | Öl 1) |
| 38 | H | $-(CH_2)_4-$ | $3-NH-COC_2H_5$ | Öl 1) |
| 39 | H | $-(CH_2)_4-$ | $3-NH-CONHCH_3$ | Öl 1) |
| 40 | H | $-(CH_2)_4-$ | $3-NH-CO_2CH_3$ | Öl 1) |

1) Chromatographiert über Kieselgel mit $CH_2Cl_2$/Aceton Mischungen.

Patentansprüche

1. Substituierte 4-Hydroxy-benzopyrane der allgemeinen Formel (I)

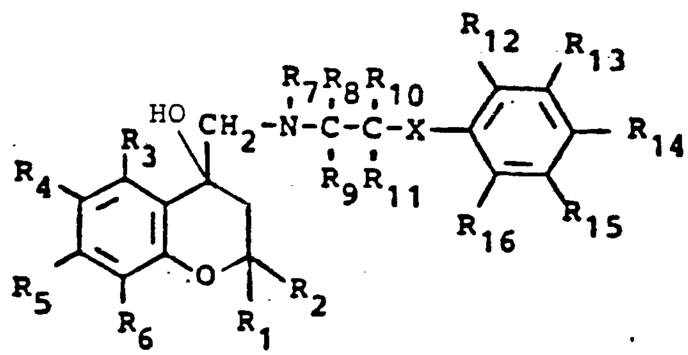

in der

$R_1$ und $R_2$ gleich oder verschieden sind und für Wasserstoff, Alkyl, Cycloalkyl, gegebenenfalls substituiertes Aryl, gegebenenfalls substituiertes Aralkyl

stehen, oder in welchen

$R_1$ und $R_2$ zusammen mit dem von ihnen eingeschlossenen Kohlenstoffatom einen carbocyclischen Ring bilden,

$R_3$,$R_4$,$R_5$ und $R_6$ gleich oder verschieden sind und für Wasserstoff, Hydroxy, Halogen, Alkyl, Cycloalkyl, gegebenenfalls substituiertes Aryl, gegebenenfalls substituiertes Aralkyl, Alkoxy, gegebenenfalls substituiertes Aryloxy und gegebenenfalls substituiertes Aralkyloxy stehen,

$R_7$, $R_8$, $R_9$, $R_{10}$ und $R_{11}$ gleich oder verschieden sind und für Wasserstoff oder Alkyl stehen,

X für eine Einfachbindung, für gegebenenfalls durch eine oder zwei Alkylgruppen substituiertes Methylen, für Sauerstoff oder für $NR_{17}$ steht, wobei

$R_{17}$ für Wasserstoff und Alkyl mit bis zu 6 Kohlenstoffatomen steht, oder

$R_{17}$ zusammen mit $R_7$ für $C_2$-$C_3$-Alkylen steht,

$R_{12}$, $R_{13}$, $R_{14}$, $R_{15}$ und $R_{16}$ gleich oder verschieden sind und jeweils für Wasserstoff, Hydroxy, Halogen, Alkyl, Cycloalkyl, Alkoxy, Aralkyloxy, Trifluormethyl, Nitro,

Cyano, die Gruppe 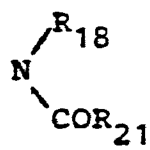 , 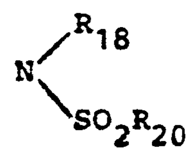 , 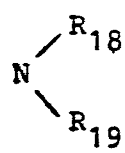 ,

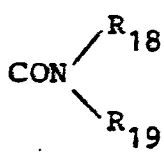 oder 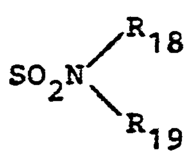 stehen,

wobei $R_{18}$ und $R_{19}$ gleich oder verschieden sind und jeweils für Wasserstoff oder gegebenenfalls substituiertes Alkyl stehen und wobei $R_{20}$ für gegebenenfalls substituiertes Alkyl steht, und wobei $R_{21}$ für Wasserstoff, gegebenenfalls

substituiertes Alkyl, gegebenenfalls substituiertes Alkoxy oder gegebenenfalls substituiertes Alkylamino steht,

und

$R_{12}$ und $R_{13}$ oder $R_{13}$ und $R_{14}$ gegebenenfalls gemeinsam jeweils für eine Alkylendioxygruppe oder die Gruppe -CH=CH-CH=CH- stehen,

oder

wobei $R_{18}$ gemeinsam mit $R_{19}$ oder gemeinsam mit $R_{20}$ oder gemeinsam mit $R_{21}$ einen 5- bis 7-gliedrigen heterocyclischen Ring bilden können, der eine Alkylenbrücke mit 2 bis 5 Kohlenstoffatomen und gegebenenfalls noch eine zusätzliche Carbonylgruppe enthält,

sowie ihre pharmazeutisch unbedenklichen Additionssalze.

2. Verbindungen der allgemeinen Formel (I) gemäß Anspruch 1 in welcher

$R_1$ und $R_2$ gleich oder verschieden für Wasserstoff, $C_1$-$C_6$-Alkyl, $C_5$-$C_7$-Cycloalkyl, Phenyl, gegebenenfalls ein- oder zweifach substituiert durch $C_1$-$C_4$-Alkyl, Halogen und/oder $C_1$-$C_4$-Alkoxy, $C_7$-$C_9$-Aralkyl, dessen Arylrest gegebenenfalls

1- oder 2-fach, substituiert ist durch $C_1$-$C_4$-Alkyl, Halogen und/oder $C_1$-$C_4$-Alkoxy, oder $R_1$ und $R_2$ bilden zusammen mit dem eingeschlossenen C-Atom des Chromanringes einen 4-bis 7-gliedrigen carbocyclischen Ring;

$R_3$ bis $R_6$ gleich oder verschieden für Wasserstoff, Hydroxy, Halogen, $C_1$-$C_6$-Alkyl, $C_5$-$C_7$-Cycloalkyl, Phenyl, gegebenenfalls 1- bis 2-fach substituiert durch $C_1$-$C_4$-Alkyl, Halogen und/oder $C_1$-$C_4$-Alkoxy, $C_7$-$C_9$-Aralkyl, dessen Arylrest gegebenenfalls 1- bis 2-fach substituiert ist durch $C_1$-$C_4$-Alkyl, Halogen und/oder $C_1$-$C_4$-Alkoxy;

$R_7$, $R_8$, $R_9$, $R_{10}$, $R_{11}$ gleich oder verschieden sind und für Wasserstoff oder $C_1$-$C_6$-Alkyl stehen,

X für eine Einfachbindung, für Methylen, gegebenenfalls ein- oder zweifach substituiert durch $C_1$-$C_4$-Alkyl, für Sauerstoff oder für $-NR_{17}$ steht, wobei $R_{17}$ Wasserstoff oder $C_1$-$C_4$-Alkyl darstellt oder $R_{17}$ zusammen mit $R_7$ einen $C_2$-Alkylenringschluß bildet,

$R_{12}$, $R_{13}$, $R_{14}$, $R_{15}$ und $R_{16}$ gleich oder verschieden sind und jeweils für Wasserstoff, Hydroxy, Chlor, Fluor, Alkyl mit 1 - 4 C-Atomen, Alkoxy mit 1 bis 3 C-Atomen, Aralkyloxy mit bis zu 8 C-Atomen, Trifluormethyl, Nitro, Cyano,

die Gruppe 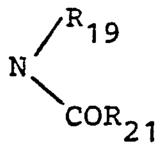 , 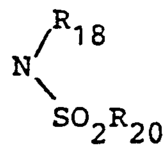 , 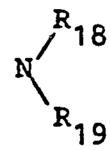 ,

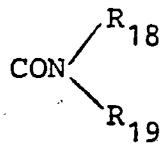 oder 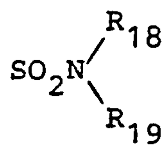 stehen, wobei $R_{18}$ und $R_{19}$ gleich oder verschieden sind und jeweils für Wasserstoff oder Alkyl mit 1 - 4 C-Atomen stehen, welches gegebenenfalls 1 bis 3 fach substituiert ist durch Halogen,

$R_{20}$ für Alkyl mit 1-6 C-Atomen steht, welches gegebenenfalls 1-3 fach substituiert ist durch Halogen, und

$R_{21}$ für Wasserstoff, Alkyl, Alkoxy oder Alkylamino mit jeweils 1-4 C-Atomen pro Alkyl- und Alkoxygruppe steht, wobei der Alkylrest gegebenenfalls 1-3 fach substituiert ist durch Halogen,

und

$R_{12}$ und $R_{13}$ oder $R_{13}$ und $R_{14}$ gegebenenfalls gemeinsam für eine Alkylendioxygruppe mit 1 oder 2 C-Atomen oder die Gruppe -CH=CH-CH=CH- stehen,

oder

wobei $R_{18}$ gemeinsam mit $R_{19}$ oder gemeinsam mit $R_{20}$ oder gemeinsam mit $R_{21}$ einen 5- oder 6-

gliedrigen heterocyclischen Ring bilden kann, der eine Alkylenbrücke mit 2, 3 oder 4 Kohlenstoffatomen enthält und gegebenenfalls noch eine zusätzliche Carbonylgruppe enthält.

3. Verbindungen der allgemeinen Formel (I) gemäß Anspruch 1 in welcher

$R_1$ und $R_2$ gleich oder verschieden sind und für Wasserstoff, $C_1$-$C_4$-Alkyl stehen oder zusammen mit dem von ihnen eingeschlossenen Kohlenstoffatom einen carbocyclischen $C_5$- oder $C_6$-Ring bilden,

$R_3$ bis $R_6$ gleich oder verschieden sind und Wasserstoff, Hydroxy, $C_1$-$C_4$-Alkyl, $C_1$-$C_4$-Alkoxy oder Chlor bedeuten,

$R_7$ bis $R_{11}$ gleich oder verschieden sind und Wasserstoff oder $C_1$-$C_4$-Alkyl darstellen,

X für eine Einfachbindung, für Sauerstoff, für Methylen oder für $-NR_{17}$ steht, wobei $R_{17}$ Wasserstoff oder $C_1$-$C_3$-Alkyl bedeutet, oder wobei $R_{17}$ zusammen mit $R_7$ einen Ethylenringschluß bildet, und

$R_{12}$ bis $R_{16}$ gleich oder verschieden sind und Wasserstoff, Chlor, Cyclohexyl, $C_1$-$C_4$-Alkyl, $C_1$-$C_3$-

Alkoxy, Aralkoxy mit 1-8 C-Atomen, Trifluormethyl, Nitro, Cyano, die Gruppe

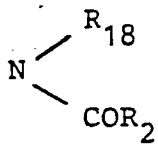 , 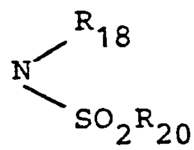 , 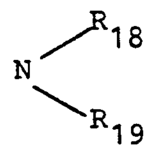 , 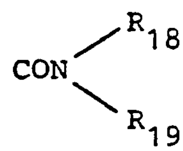 ,

oder 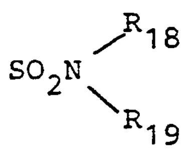 bedeuten, wobei $R_{12}$ und $R_{13}$

oder $R_{13}$ und $R_{14}$ gegebenenfalls zusammen jeweils eine Methylendioxygruppe oder eine -CH=CH-CH=CH-Gruppe bilden, und wobei $R_{18}$ und $R_{19}$ gleich oder verschieden sind und jeweils für Wasserstoff oder gegebenenfalls durch Fluor oder Chlor substituiertes Alkyl mit 1-4 C-Atomen stehen und wobei $R_{20}$ für gegebenenfalls durch Fluor oder Chlor substituiertes Alkyl mit 1-4 C-Atomen steht und wobei $R_{21}$ für Wasserstoff, gegebenenfalls durch Fluor oder Chlor substituiertes Alkyl mit 1-4 C-Atomen, Alkoxy oder Alkylamino mit jeweils 1-2 C-Atomen steht.

4. Verfahren zur Herstellung von Verbindungen der allgemeinen Formel (I)

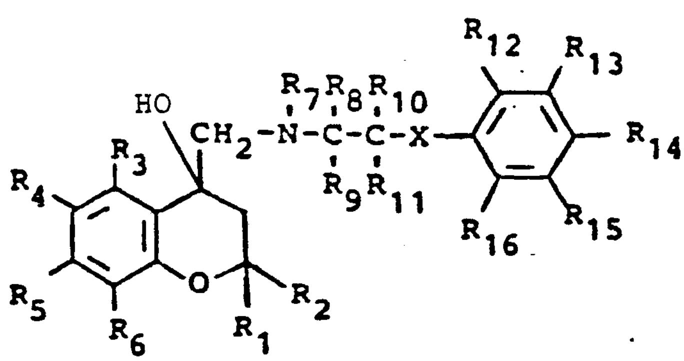

in der

$R_1$ und $R_2$ gleich oder verschieden sind und für Wasserstoff, Alkyl, Cycloalkyl, gegebenenfalls substituiertes Aryl, gegebenenfalls substituiertes Aralkyl,

stehen, oder in welchen

$R_1$ und $R_2$ zusammen mit dem von ihnen eingeschlossenen Kohlenstoffatom einen carbocyclischen Ring bilden,

$R_3$,$R_4$,$R_5$ und $R_6$ gleich oder verschieden sind und für Wasserstoff, Hydroxy, Halogen, Alkyl, Cycloalkyl, gegebenenfalls substituiertes Aryl, gegebenenfalls substituiertes Aralkyl, Alkoxy, gegebenenfalls substituiertes Aryloxy und gegebenenfalls substituiertes Aralkyloxy stehen,

$R_7$,$R_8$,$R_9$,$R_{10}$ und $R_{11}$ gleich oder verschieden sind und für Wasserstoff oder Alkyl stehen,

X für eine Einfachbindung, für gegebenenfalls durch eine oder zwei Alkylgruppen substituiertes Methylen, für Sauerstoff oder für $NR_{17}$ steht, wobei

$R_{17}$ für Wasserstoff und Alkyl mit bis zu 6 Kohlenstoffatomen steht, oder

$R_{17}$ zusammen mit $R_7$ für $C_2$-$C_3$-Alkylen steht,

$R_{12}$, $R_{13}$, $R_{14}$, $R_{15}$ und $R_{16}$ gleich oder verschieden sind und jeweils für Wasserstoff, Hydroxy, Halogen, Alkyl, Cycloalkyl, Alkoxy, Aralkyloxy. Trifluormethyl, Nitro, Cyano, die Gruppe

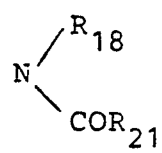 , 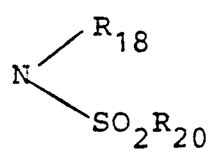 , 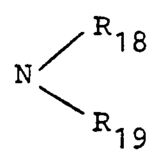 ,

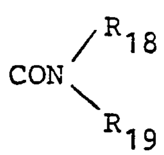 oder 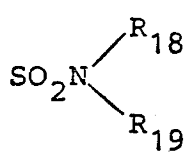 stehen,

wobei $R_{18}$ und $R_{19}$ gleich oder verschieden sind und jeweils für Wasserstoff oder gegebenenfalls substituiertes Alkyl stehen und wobei $R_{20}$ für gegebenenfalls substituiertes Alkyl steht, und wobei $R_{21}$ für Wasserstoff, gegebenenfalls substituiertes Alkyl, gegebenenfalls substituiertes Alkoxy oder gegebenenfalls substituiertes Alkylamino steht,

und

$R_{12}$ und $R_{13}$ oder $R_{13}$ und $R_{14}$ gegebenenfalls gemeinsam jeweils für eine Alkylendioxygruppe oder die Gruppe -CH=CH-CH=CH- stehen,

oder

wobei $R_{18}$ gemeinsam mit $R_{19}$ oder gemeinsam mit $R_{20}$ oder gemeinsam mit $R_{21}$ einen 5- bis 7-gliedrigen heterocyclischen Ring bilden können, der eine Alkylenbrücke mit 2 bis 5 Kohlenstoffatomen und gegebenenfalls noch eine zusätzliche Carbonylgruppe enthält,

sowie ihrer pharmazeutisch unbedenklichen Additionssalze, dadurch gekennzeichnet, daß man

A) Amine der Formel (II)

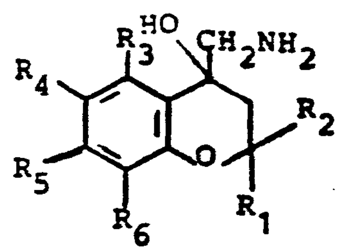

in welcher

$R_1, R_2, R_3, R_4, R_5$ und $R_6$ die oben angegebene Bedeutung haben,

mit Carbonylverbindungen der Formel (III)

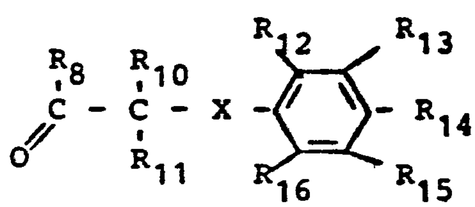

in welcher

$R_8$ bis $R_{16}$ die oben angegebene Bedeutung haben, mit der Maßgabe, daß X nicht für $NR_{17}$ steht,

in Gegenwart von reduzierenden Agentien umsetzt,

oder

B) Verbindungen der Formel (IV)

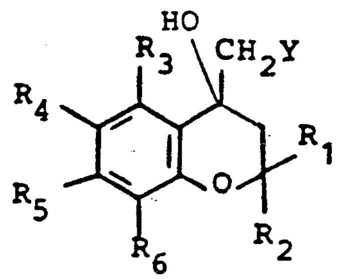

(IV) oder

gegebenenfalls nach Isolierung der durch Abspaltung von HY entstehenden Verbindung der Formel (IVa)

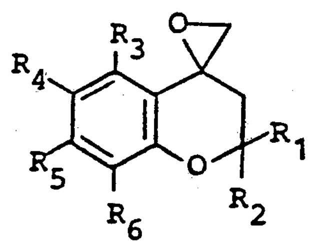

(IVa)

in welcher

$R_1$, $R_2$, $R_3$, $R_4$, $R_5$ und $R_6$ die oben angegebene Bedeutung haben, und

Y für nucloephil austauschbare Gruppen, wie Alkyl- oder Aryl-substituierte Sulfonyloxygruppen, Brom oder Chlor steht,

mit den Aminen der Formel (V)

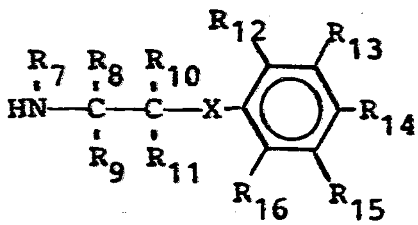

(V)

in Gegenwart eines säurebindenden Mittels umsetzt.

5. Verfahren gemäß Anspruch 4, dadurch gekennzeichnet, daß man bei der Verfahrensvariante A) als reduzierende Agentien komplexe Metallhydride oder katalytisch angeregten Wasserstoff einsetzt und bei der Verfahrensvariante B) als säurebindende Mittel Metallhydroxide, Carbonate oder Stickstoffbasen einsetzt.

6. Verfahren gemäß Anspruch 4, dadurch gekennzeichnet, daß man die Umsetzungen bei Temperaturen zwischen -50 und +150°C durchführt.

7. Arzneimittel enthaltend mindestens eine Verbindung der allgemeinen Formel (I) gemäß Anspruch 1.

8. Verfahren zur Herstellung von Arzneimitteln, dadurch gekennzeichnet, daß man mindestens eine Verbindung der allgemeinen Formel (I) gemäß Anspruch 1 unter Verwendung von inerten Hilfs- und Trägerstoffen in eine geeignete Applikationsform überführt.

9. Verwendung von VerLindungen der allgemeinen Formel (I) gemäß Anspruch 1 bei der Bekämpfung von Erkrankungen.

10. Verwendung von Verbindungen der allgemeinen Formel (I) gemäß Anspruch 1 bei der Bekämpfung von Kreislauferkrankungen.